# EUROPEAN PATENT APPLICATION

(11) **EP 4 052 719 A1**
(43) Date of publication of application: **07.09.2022**
(21) Application number: 20902629.3
(22) Date of filing: 18.12.2020
(51) Int. Cl.: A61K 36/756, A61P 17/00

(54) **TRADITIONAL CHINESE MEDICINE COMPOSITION FOR TREATING EGFR-TKIS RELATED SKIN RASH AND USE THEREOF**

(30) Priority: 20.12.2019 CN 201911327934
(71) Applicant: Yangtze River Pharmaceutical Group Guangzhou Hairui Pharmaceutical Co., Ltd., Guangzhou, Guangdong 510663 (CN); Yangtze River Pharmaceutical Group Co., Ltd., Taizhou, Jiangsu 225321 (CN); Yangtze River Pharmaceutical Group Jiangsu Longfengtang Traditional Chinese Medicine Co., Ltd., Taizhou, Jiangsu 225327 (CN)
(72) Inventor: LIN, Lizhu, Guangzhou, Guangdong 510405 (CN)
(74) Representative: Petraz, Gilberto Luigi
(86) International application number: PCT/CN2020/137615
(87) International publication number: WO 2021/121388

(57) **Abstract**

The present disclosure provides a traditional Chinese medicine composition for treating EGFR-TKIs related skin rash, including therapeutically effective amounts of the following components: *Lonicera japonica, Taraxacum mongolicum, Sophora flavescens, Kochiae fructus, dictamni cortex, moutan cortex, Phellodendri Chinensis Cortex,* and *Menthae Haplocalycis Herba.* The use of the traditional Chinese medicine composition for treating EGFR-TKIs related skin rash has a certain effect on the treatment of EGFR-TKIs related skin rash and paronychia. By coordinating various raw materials with each other, the medicine effect can be exerted to the greatest extent, thereby obviously improving the life quality of a patient and prolonging the survival time of the patient. In addition, the traditional Chinese medicine composition has the advantages of no toxic side effects, low treatment cost and the like.

## Description

The present disclosure claims the priority of a prior application with the patent application number of 201911327934.5 and entitled "TRADITIONAL CHINESE MEDICINE COMPOSITION FOR TREATING EGFR-TKIS RELATED SKIN RASH AND USE THEREOF", which was filed to the China National Intellectual Property Administration on December 20th, 2019. The entire content of the prior application is incorporated in this application by reference.

### TECHNICAL FIELD

The present disclosure belongs to the technical field of traditional Chinese medicine, and in particular relates to a traditional Chinese medicine composition for treating EGFR-TKIs related skin rash and use thereof.

### BACKGROUND

The 2018 Global Cancer Annual Report has shown that among the 18 million new cancer cases and 9.6 million cancer death cases in the world, in China the number of new cancer cases has reached 3.804 million and the number of death cases has reached 2.296 million, ranking first in the world. Molecular targeted therapy, as a novel treatment means, takes a cancer-related molecule as a target, and targets an effective component such as a drug, an antibody and the like to a cancer cell and a component related thereto, so as to achieve the purpose of treating cancer. In Guidelines for Lung Cancer 2018 of the Chinese Society of Clinical Oncology (CSCO), Gefitinib, Icotinib, Erlotinib and Afatinib are recommended for the first-line treatment of stage IV driver gene positive NSCLC (type I evidence).

When such targeted drug therapy is applied in clinic, it is often accompanied with various skin-related side effects, which are mostly manifested as acne-like skin rash and paronychia, paratrichosis, itching and xerodermia. In addition, it is related to epidermal growth factor receptor (EGFRI), that is, it is known as a PRIDE syndrome (a common skin response of the EGFRIs is called the PRIDE syndrome, wherein P" refers to papulopustule and paronychia, "R" refers to regulation and change of hair growth, "I" refers to itching, "D" refers to dryness, and "E" refers to EGFRIs). In the phase III clinical study of epidermal growth factor receptor tyrosine kinase inhibitors (EGFR-TKIs), the incidence of skin rash/acne-like skin rash was 15.5%-89.1%. About 1 to 2 weeks after the start of treatment, itching/painful maculopapules and purulent herpes were developed in areas rich in sebaceous glands, such as the face, chest and back, of the patients, one third of the patients had obvious related itching and scorching hot, and 38% of the patients had secondary skin infections, all of which led to the decline of the patients' quality of life.

For the management of EGFR-TKIs related skin rash at home and abroad, there is not enough therapeutic research evidence to support it, and most of the evidences are based on preventive medication research. At home and abroad, antibiotics and hormones are suggested to be used as symptomatic treatment in therapy, but the actual clinical efficacy is not clear, and meanwhile adoption of a systemic treatment solution may affect the anti-tumor autoimmune response level of the patient to reduce the efficacy of EGFR-TKIs. Furthermore, for patients with moderate to severe skin rash, they often choose to reduce dosage, take medicine intermittently or stop taking medicine because of intolerance, which limits these patients from getting benefits from the targeted therapy.

### SUMMARY

The present inventor has found through research that EGFR-TKIs related skin rash belongs to the category of "drug toxicity rash", "damp toxin sore" and "wind toxin swelling" in the category of traditional Chinese medicine. Based on the knowledge and clinical experience of predecessors, modern physicians have concluded that the basic pathogenesis of skin rash caused by EGFR-TKIs is that yin deficiency and blood dryness are in the body, and toxic and pathogenic factors are concentrated outside, so it is advisable to nourish yin and moisten dryness in the treatment to help the root vacuity, and then to relieve its tip repletion by ventilating the lung, clearing away heat, cooling blood, and removing blood stasis depending on different stages of pathogenic factors. Therefore, "clearing away heat and cooling blood, eliminating dampness and relieving itching" is proposed as a basic plan for treating skin rash caused by EGFR-TKIs, and a topical prescription for eliminating skin rash and relieving itching is drawn up. The main indications of this prescription are clearing away heat and cooling blood, removing blood stasis and relieving itching. The prescription uses *Lonicera japonica* as the monarch drug to clear away heat and toxic materials and disperse wind heat; *moutan cortex* as a ministerial drug to cool blood and promote blood circulation; *Sophora flavescens, Taraxacum mongolicum* and *Phellodendri Chinensis Cortex* as assistant drugs to clear away heat, eliminate dampness and detoxicate, kill insects and stop itching; *Kochiae fructus, dictamni cortex* and *Menthae Haplocalycis Herba* as conductant drugs to clear away heat, eliminate dampness, dispel wind and stop itching. Moreover, Meta-analysis shows that intervention of this traditional Chinese medicine prescription can significantly improve the EGFR-TKIs related skin rash and improve the life quality of the patients, while not affecting the curative effect of EGFR-TKIs.

The present disclosure provides a traditional Chinese medicine composition, the traditional Chinese medicine composition is prepared according to a certain ratio based on a prescription theory, preferably the traditional Chinese medicine composition for treating EGFR-TKIs related skin rash, including therapeutically effective amounts of the following components: *Lonicera japonica, Taraxacum mongolicum, Sophora flavescens, Kochiae fructus, dictamni cortex, moutan cortex, Phellodendri Chinensis Cortex,* and *Menthae Haplocalycis Herba.*

Introduction of traditional Chinese medicine components:
*Lonicera japonica:* is the dried flower bud or flower with initial bloom of *Lonicera japonica Thunb., Lonicera L.hypoglaucaMiq., L.confucaDC.* or *L.dasystylaRehd..* It has the effects of clearing away heat and toxic materials and dispersing wind heat. It can be used for indications of swollen welling-abscess and clove sores, wind-heat common cold, incipient febrile diseases, heat toxin and bloody dysentery.

*Taraxacum mongolicum:* it is the dried whole plant of *Taraxacummongolicum Hand. -Mazz., T. sinicumKitag.,* or several plants of the same genus in Compositae. It has the effects of clearing away heat and toxic materials, reducing swelling and resolving hard mass, promoting diuresis and treating stranguria. It can be used for indications of carbuncle, swelling and furuncle, acute mastitis and internal carbuncle, stranguria due to damp-heat, jaundice due to damp heat, removing liver-fire for improving eyesight.

*Sophora flavescens:* is the dried root of *Sophoraflavescens Ait* of Leguminosae. It has the effects of clearing away heat and eliminating dampness, killing insects and inducing diuresis. It can be used for indications of damp-heat diarrhea, bloody stool, jaundice, damp-heat leukorrhagia, pudendal swelling and pruritus, eczema and wet sores, skin pruritus, scabies, difficult urination caused by damp-heat.

*Kochiae fructus:* is the mature fruit of *Kochiascoparia (L.) Schrad* of Polygonaceae. It has the effects of inducing diuresis and treating stranguria, clearing away heat and eliminating dampness, and stopping itching. It can be used for indications of stranguria, pruritus vulvae and leukorrhagia, rubella and eczema.

*Dictamni cortex:* is the dried root bark of *DictamnusdasycarpusTurcz.* of Rutaceae. It has the effects of clearing away heat and eliminating dampness, dispelling wind and detoxicating. It can be used for indications of damp-heat sore toxin, eczema, scabies, stranguria due to damp-heat and wind-damp-heat bi diseases.

The *moutan cortex:* is the dried *moutan cortex* of Ranunculaceae. It has the effects of clearing heat, cooling blood, promoting blood circulation and removing blood stasis. It can be used for indications of heat toxin with skin eruption, bleeding from five aperture or subcutaneous tissue due to blood heat, impairment of yin due to febrile diseases, fever due to yin deficiency, night fever abating at dawn, hectic fever without sweating, amenorrhea due to stagnation of blood and dysmenorrhea, traumatic injury, carbuncle, swelling and sores.

*Phellodendri Chinensis Cortex.:* is the dried bark of *PhellodendronchinenseSchneid.* of rutaceae. It has the effects of clearing away heat and eliminating dampness, discharging fire and detoxicating, and removing hectic fever. It can be used for indications of damp-heat leukorrhagia, stranguria due to damp-heat, damp-heat diarrhea, jaundice, beriberi due to damp-heat, flaccidity syndrome, hectic fever and consumptive fever, night sweats, spermatorrhea, sores ulceration and swelling toxin, eczema and itching.

*Menthae Haplocalycis Herba:* is the dried whole plant of a plant with the Latin name of *Mentha haplocalyx Briq,* and the local name of "Yindan grass" of labiatae, and other plants of the same genus. It has the effects of dispelling wind and clearing heat. It can be used for indications of wind-heat type common cold, headache, hot eyes, swelling and pain in throat, toothache and itchy skin.

The traditional Chinese medicines used in the present disclosure can be purchased from general Chinese pharmacies, and their specifications conform to the standard of Chinese Pharmacopoeia.

As a preferred solution of the present disclosure, the composition further includes the following components in parts by weight:
5-40 parts of *Lonicera japonica,* 5-50 parts of *Taraxacum mongolicum,* 3-50 parts of *Sophoraflavescens,* 5-50 parts of *Kochiae fructus,* 3-50 parts of *dictamni cortex,* 5-50 parts of *moutan cortex,* 5-50 parts of *Phellodendri Chinensis Cortex,* and 2-30 parts of *Menthae Haplocalycis Herba.*

According to a preferred embodiment of the present disclosure, the parts by weight of *Lonicera japonica* can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40;

The parts by weight of *Taraxacum mongolicum* can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

The parts by weight of *Sophora flavescens* can be 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

The parts by weight of *Kochiae fructus* can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

The parts by weight of *dictamni cortex* can be 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

The parts by weight of the *moutan cortex* can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50;

The parts by weight of *Phellodendri Chinensis Cortex.* can be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50; and

The parts by weight of *Menthae Haplocalycis Herba* can be 2, 3, 4, 5, 6, 7, 8, 9, 10, 11, 12, 13, 14, 15, 16, 17, 18, 19, 20, 21, 22, 23, 24, 25, 26, 27, 28, 29 or 30.

As a most preferred solution of the present disclosure, the composition further includes the following components in parts by weight:
15 parts of *Lonicera japonica,* 15 parts of *Taraxacum mongolicum,* 9 parts of *Sophora flavescens,* 15 parts of *Kochiae fructus,* 10 parts of *dictamni cortex,* 12 parts of *moutan cortex,* 12 parts of *Phellodendri Chinensis Cortex,* and 6 parts of *Menthae Haplocalycis Herba.*

The present disclosure also provides use of the traditional Chinese medicine composition as described above, e.g., the traditional Chinese medicine composition for treating EGFR-TKIs related skin rash, in preparation of a formulation for treating EGFR-TKIs related skin rash.

The present disclosure also provides use of the traditional Chinese medicine composition as described above, e.g., the traditional Chinese medicine composition for treating EGFR-TKIs related skin rash, in preparation of a formulation for treating EGFR-TKIs related paronychia.

Preferably, the formulation is a liniment, water aqua, decoction, granule, paste, mistura, aerosol, gel, ointment, facial mask, cream or unguent.

The present disclosure also provides a method for treating EGFR-TKIs related skin rash or paronychia, including administering the traditional Chinese medicine composition to a patient in need thereof, such as a human.

After 4-8 weeks of EGFR-TKI treatment, changes will occur in the patient's fingernails or toenails, usually red and swollen and pain begin to appear from the edge of the root of the finger(toe)nails, and then symptoms such as inflammation, ulcer, occurrence of suppurative granulation tissues, etc., are gradually developed in nail grooves on both sides, which enables the finger(toe)nails to be embedded, causing mobility inconvenience of the patient. The main clinical manifestations of paronychia are as follows: (1) initially starting of skin rash from periungual skin; (2) formation of granulation tissues; and (3) infection with gram-positive bacteria (G+), gram-negative bacteria (G-) and *Candida albicans* as found on the smear. After the Chinese medicinal composition of the present disclosure is prepared into an unguent and applied to the affected part for 2 weeks, the symptoms are improved.

### Beneficial effects of the present disclosure

The use of the traditional Chinese medicine composition for treating EGFR-TKIs related skin rash has a certain effect on the treatment of EGFR-TKIs related skin rash and paronychia. By coordinating various raw materials with each other, the medicine effect can be exerted to the greatest extent, thereby obviously improving the life quality of a patient and prolonging the survival time of the patient. In addition, the traditional Chinese medicine composition has the advantages of no toxic side effects, low treatment cost and the like.

### BRIEF DESCRIPTION OF DRAWINGS

FIG. 1 is a schematic diagram of a change trend of a part C score of a WoMo scale in treating EGFR-TKIs related skin rash with the traditional Chinese medicine composition of the present disclosure.
FIG. 2 is a schematic diagram of a change trend of a total score of the WoMo scale in treating EGFR-TKIs related skin rash with the traditional Chinese medicine composition of the present disclosure.
FIG. 3 is a comparison of EGFR-TKIS related paronychia before and after treatment in case 1 of Example 4 (a is before treatment; and b is after treatment).
FIG. 4 is a comparison of EGFR-TKIS related paronychia before and after treatment in case 2 of Example 4 (a is before treatment; and b is after treatment).
FIG. 5 is a comparison of EGFR-TKIS related skin rash before and after treatment in case 1 of Example 5 (a is before treatment; and b is after treatment).
FIG. 6 is a comparison of EGFR-TKIS related skin rash before and after treatment in case 2 of Example 5 (a is before treatment; and b is after treatment).
FIG. 7 is a comparison of EGFR-TKIS related skin rash before and after treatment in case 3 of Example 5 (a is before treatment; and b is after treatment).

### DETAILED DESCRIPTION OF EMBODIMENTS

In order to show the technical solution, objective and advantages of the present disclosure more concisely and clearly, the present disclosure will be further described in detail below with reference to specific examples and accompanying drawings of the present disclosure.

Example 1: This example provided a traditional Chinese medicine composition for treating EGFR-TKIs related skin rash.

15 g of *Lonicera japonica,* 15 g of *Taraxacum mongolicum,* 9 g of *Sophora flavescens,* 15 g of *Kochiae fructus,* 10 g of *dictamni cortex,* 12 g of the *moutan cortex,* 12 g of *Phellodendri Chinensis Cortex,* and 6 g of *Menthae Haplocalycis Herba* (added lately). The whole prescription was 94 g, which was decocted in water and concentrated to 50 ml, containing 1.88 g crude drug per ml.

Example 2: This example provided a traditional Chinese medicine composition for treating EGFR-TKIs related skin rash.

5 parts of *Lonicera japonica,* 5 parts of *Taraxacum mongolicum,* 3 parts of *Sophora flavescens,* 5 parts of *Kochiae fructus,* parts of *dictamni cortex,* 5 parts of *moutan cortex,* 5 parts of *Phellodendri Chinensis Cortex,* and 2 parts of *Menthae Haplocalycis Herba.*

Example 3: This example provided a traditional Chinese medicine composition for treating EGFR-TKIs related skin rash.

15 parts of *Lonicera japonica,* 10 parts of *Taraxacum mongolicum,* 8 parts of *Sophora flavescens,* 10 parts of *Kochiae fructus,* 12 parts of *dictamni cortex,* 6 parts of *moutan cortex,* 8 parts of *Phellodendri Chinensis Cortex,* and 10 parts of *Menthae Haplocalycis Herba.*

Example 4: This example provided a traditional Chinese medicine composition for treating EGFR-TKIs related skin rash.

20 parts of *Lonicera japonica,* 18 parts of *Taraxacum mongolicum,* 20 parts of *Sophora flavescens,* 20 parts of *Kochiae fructus,* 25 parts of *dictamni cortex,* 10 parts of *moutan cortex,* 10 parts of *Phellodendri Chinensis Cortex,* and 12 parts of *Menthae Haplocalycis Herba.*

Example 5: This example provided a traditional Chinese medicine composition for treating EGFR-TKIs related skin rash.

40 parts of *Lonicera japonica,* 50 parts of *Taraxacum mongolicum,* 50 parts of *Sophora flavescens,* 50 parts of *Kochiae fructus,* 50 parts of *dictamni cortex,* 50 parts of *moutan cortex,* 50 parts of *Phellodendri Chinensis Cortex,* and 30 parts of *Menthae Haplocalycis Herba.*

Example 6: This example provided a method for preparing a traditional Chinese medicine composition for treating EGFR-TKIs related skin rash, which included the following specific steps:
(1) the aforementioned composition was weighed, added with 10 times the volume of water for soaking, decocted for 40-60 minutes, and then filtered to obtain a first medical solution;
(2) the dregs of decoction obtained in the first step was added with additional 10 times the volume of water, decocted for 30-40 minutes, and then filtered to obtain a second medical solution; and
(3) the filtrates obtained in step (1) and step (2) were combined and centrifuged, and the filtrate was concentrated to obtain a water decoction.

In the aforementioned preparation steps, after the twice decocting for a specified time, in order to avoid some components from losing due to precipitation after the water decoction was cooled, the medical solutions obtained in the two steps were filtered while they were hot.

(4) The decoction in step (3) was to heat and boil with high fire first, and then slowly decoct with mild fire to keep the solution in a slightly boiling state, which could not only reduce the volatilization of water, prevent the solution from drying and scorching, but also promote the extracting of effective components in medicinal materials.

(5) As a preferred protocol, the filtrate was concentrated to a filtrate concentration of 1.88 g/ml (i.e., every 1 ml of the concentrated solution contained 1.88 g of a decoction product of drugs), which was beneficial to control the volume of subsequent administration and exert a better pharmaceutical effect.

### Example 7

The traditional Chinese medicine composition prepared in Example 1 was used for treating EGFR-TKIs related skin rash, specifically as follows:

### 1. Treatment protocol

(1) Characteristics of population to be treated: 40 cases of patients who took EGRF-TKI and developed related skin rash. The average age of the patients was 54.70 ± 12.00 years old, with the youngest being 30 years old and the oldest being 82 years old, wherein there were 25 males and 15 females. 32 cases (80.00%) were enrolled into stage IV, and 8 cases (20.00%) were enrolled into stage III. Mutation of exon 19 occurred in 12 cases (30.00%), mutation of L858R occurred in 19 cases (47.50%), and the details of 9 cases (22.50%) were unknown. Targeted drugs included Alfatinib in 15 cases (37.50%), Erlotinib in 7 cases (17.50%), and Gefitinib in 7 cases (17.50%), Icotinib in 5 cases (12.50%), Osimertinib in 4 cases (10.00%) and Dacomitinib in 2 cases (5.00%). The time from medication to occurrence of skin rash was 13.03 ± 15.18 days.

**Table 1. Description of study population**

| | |
|---|---|
| Number of persons (cases) | 40 |
| Age | 54.70 ± 12.00 (30.00-82.00) |
| (mean ± SD; min-max, years old) | |
| Gender (case, %) | |
| Male | 25 (62.50%) |
| Female | 15 (37.50%) |
| Stages (case, %) | |
| Stage IV | 32 (80.00%) |
| Stage III | 8 (20.00%) |
| Gene detection result (case, %) | |
| Exon 19 deletion mutation | 16 (40.00%) |
| L858R mutation | 24 (60.00%) |
| Targeted drug (case, %) | |
| Afatinib | 15 (37.50%) |
| Erlotinib | 7 (17.50%) |
| Gefitinib | 7 (17.50%) |
| Icotinib | 5 (12.50%) |
| Osimertinib | 4 (10.00%) |
| Dacomitinib | 2 (5.00%) |
| Time from medication to skin rash (days) | 13.03 ± 15.18 |

(2) Using method: external use, applying to the skin of patients with skin rash. 50 ml was administrated each time for twice a day, with 30 minutes each time, by externally applying onto the affected part or soaking the affected part. Each subject was given an externally applied facial mask of a decoction of skin rash-eliminating and itching-stopping prescription on his/her face, once in the morning and once in evening of a day, for 2 weeks.

### 2. Evaluation indicators

(1) Main research outcomes: a WoMo scale was used for evaluating the effectiveness of the skin rash-eliminating and itching-stopping prescription in treating EGFR-TKI related skin rash. It was compared whether there was any difference in the decrease of the score of the WoMo scale before and after treatment. In addition, a standard error of 1.96 for the difference before and after treatment was set as the minimally important clinical difference (MICD).

(2) Adverse reactions: the adverse events occurred during the research were recorded.

### 3. Statistical analysis

A Chi-square test was used for all counting data, wherein pearson Chi-square was used when the expected count < 5 was no more than 20% and the minimum expected count ≥ 1; continuity correction was used when the expected count < 5 was more than 20% and the minimum expected count was ≥ 1; and a fisher or likelihood ratio method was used when the minimum expected count < 1 or n < 40. Measurement data were expressed by mean ± standard error (mean ± SD), and all measurement data used a homogeneity test for variance. On the contrary, a Mann-Whitney U test or t' test was used for inter-group comparison, and a Wilcoxon signed-rank test or Wilcoxon signed rank test was used for intra-group comparison.

The outcome indicators of the research included the score change of the WoMo scale, such as a baseline (T0), after the first week of intervention (T1) and after the second week of intervention (T2), which belonged to replicate measurement data. A Generalized Estimation equation (GEE) is used for calculation of the replicate measurements.

p < 0.05 was considered to be statistically difference. All confidence intervals were bilateral 95% confidence intervals. The differences between the two groups were compared, and all statistical hypotheses were 2-side tests. Empowerstats (www.empowerstats.com; X&Y solutions Inc., Boston, MA) and R (http://www.R-project.org) would be used for analysis.

### 4. Treatment results

(1) The traditional Chinese medicine composition of the present disclosure could decrease the score of the WoMo scale

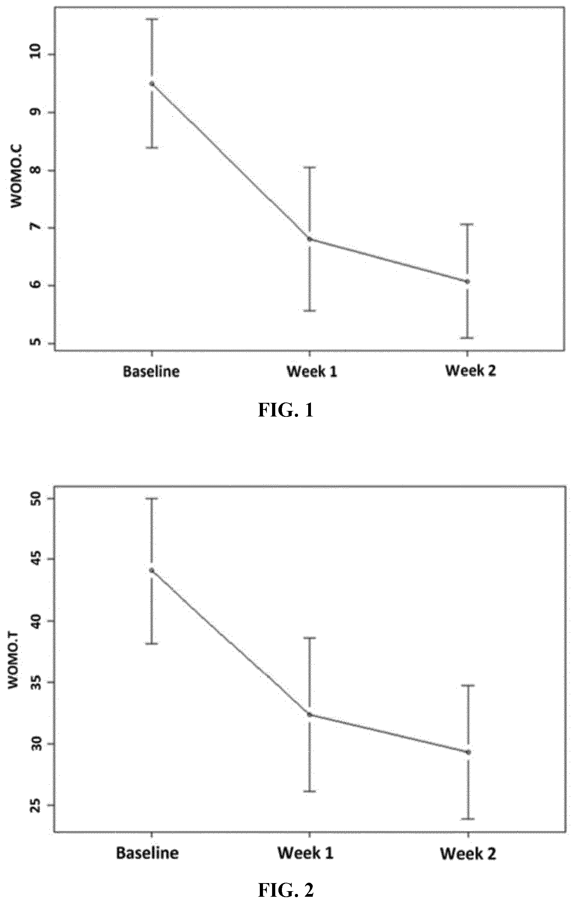

The GEE was used for comparing the score changes of the WoMo scale before and after two weeks of treatment on the patients. As could be seen from FIG. 1 and Table 2, in the analysis, a baseline time was taken as the baseline of dependent variables, and for comparison at different time points, the partial regression coefficients of the week 1 (Time 1) and the week 2 (Time 2) were -2.4809 and -3.5863, respectively, which were both statistically significant (both with p values < 0.0001). It showed that over time, the part C score of the WoMo scale has a significant decreasing trend, suggesting that the treatment was effective.

**Table 2. Estimation of fixed effect parameters of linear mixed effect model (part C of WoMo scale)**

| | Estimated Value | Standard error | Degree of Freedom | T value | P value | 95% CI. low | 95% CI. upp |
|---|---|---|---|---|---|---|---|
| Intercept | 9.5142 | 0.4878 | 48 | 19.5045 | <0.0001 | 8.5581 | 10.4703 |
| Week 1 | -2.4809 | 0.5673 | 48 | -4.3732 | <0.0001 | -3.5928 | -1.369 |
| Week 2 | -3.5863 | 0.5365 | 48 | -6.6844 | <0.0001 | -4.6379 | -2.5347 |

As could be seen from FIG. 2 and Table 3, when the GEE was used for analyzing the total score of the WoMo scale, in the analysis, a baseline time was taken as the baseline of dependent variables, and for comparison at different time points, the partial regression coefficients of the week 1 (Time 1) and the week 2 (Time 2) were -10.4973 and -15.5509, respectively, which were both statistically significant (both with p values < 0.0001). It showed that over time, the part C score of the WoMo scale has a significant decreasing trend, suggesting that the treatment was effective.

**Table 3. Estimation of fixed effect parameters of linear mixed effect model (total score of WoMo scale)**

| | Estimated Value | Standard error | Degree of Freedom | T value | P value | 95% CI. low | 95% CI. upp |
|---|---|---|---|---|---|---|---|
| Intercept | 44.4161 | 2.5721 | 47 | 17.2682 | <0.0001 | 39.3747 | 49.4575 |
| Week 1 | -10.4973 | 2.4437 | 47 | -4.2957 | <0.0001 | -15.2869 | -5.7077 |
| Week 2 | -15.5509 | 2.3103 | 47 | -6.7312 | <0.0001 | -20.079 | -11.0228 |

### (2) Adverse reactions: no adverse drug reactions were found during the treatment.

### Example 8

The traditional Chinese medicine composition prepared in Example 1 was used for treating EGFR-TKIs related paronychia, specifically as follows:

### 1. Treatment protocol

(1) Characteristics of population to be treated: 16 cases of patients who took EGRF-TKI and developed paronychia. The average age of the patients was 53.81 ± 11.99 years old, wherein there were 8 males and 8 females. 12 cases (75.00%) were enrolled into stage IV, and 4 cases (25.00%) were enrolled into stage IIIB. Mutation of exon 19 occurred in 10 cases (62.50%), L858R mutation occurred in 5 cases (31.25%), and 861Q mutation occurred in 1 case (6.25%). Among them, 15 cases were treated with Afatinib, and 1 case was treated with Gefitinib.

**Table 4. Description of study population**

| Number of persons (cases) | 16 |
|---|---|
| | Mean ± SD |
| Age (years old) | 53.81 ± 11.99 |
| | N (%) |
| Gender (case, %) | |
| Female | 8 (50.00%) |
| Male | 8 (50.00%) |
| Gene mutation | |
| EGFR Exon 19 | |
| Deletion | 10 (62.50%) |
| L858 R. | 5(31.2.5%) |
| 861Q | 1 (6.2.5%) |
| TNM staging | |
| IV | 12 (75.00%) |
| III B | 4 (25.00%) |
| Used drug | |
| Afatinib | 15 (93.75%) |
| Gefitinib | 1 (6.25%) |

(2) Using method: external use, wherein 50 ml was administrated each time for twice a day, with 30 minutes each time, by externally applying onto the affected part or soaking the affected part. The administration was conducted once in the morning and once in evening of a day, for 2 weeks.

### 2. Evaluation indicators

(1) Main research outcomes: the grading of paronychia referred to the CTCAE 5.0 standard grading issued by NCI in 2017. See Table 5 for details. It was set that it was effective if it was decreased by one grade after treatment, it had a remarkable effect if it was decreased by two grades, and it was recovered if the paronychia was disappeared.

**Table 5. Grading standard of paronychia**

| Grade 1 | Grade 2 | Grade 3 | Grade 4 | Grade 5 |
|---|---|---|---|---|
| Nailfold edema or erythema; damaged stratum corneum | In need of local treatment; oral administration of a drug (e.g., antibiotics, antifungal, antiviral therapy) for treatment; nailfold edema or painful erythema; nail shedding or nail plate separation; affecting daily life and instrumental activities | In need of surgical treatment; intravenous administration of antibiotics for treatment; affecting self-rational daily life activities | - | - |

(2) Adverse reactions: the adverse events occurred during the research were recorded.

### 3. Statistical analysis

(1) A Chi-square test was used for all counting data, wherein pearson Chi-square was used when the expected count < 5 was no more than 20% and the minimum expected count ≥ 1; continuity correction was used when the expected count < 5 was more than 20% and the minimum expected count was ≥ 1; and a fisher or likelihood ratio method was used when the minimum expected count < 1 or n < 40. Measurement data were expressed by mean ± standard error (mean ± SD).

p < 0.05 was considered to be statistically significant. All confidence intervals were bilateral 95% confidence intervals. the differences between the two groups were compared, and all statistical hypotheses were 2-side tests. Empowerstats (www.empowerstats.com; X&Y solutions Inc., Boston, MA) and R (http://www.R-project.org) would be used for analysis.

### 4. Treatment results

(1) The therapeutic effect on paronychia after 2 weeks of external application of the skin rash-eliminating and itching-stopping prescription

Results were as shown in Table 6, and 16 persons with paronychia belonged to CTCAE 5.0 grade 2 (100.00%). After treatment, 3 persons (18.750%) were recovered (grade 0), and 13 persons (81.250%) were reduced to grade 1 according to the CTCAE 5.0 grading. The composition was effective in 16 persons, with an effective rate of 100.00%; and had remarkable effect/recovery effect in 3 persons, with the rate of remarkable effect of 18.750%.

There was a statistical difference in CTCAE staging before and after treatment (p = 0.012).

**Table 6. Therapeutic effect of the traditional Chinese medicine composition of the present disclosure on paronychia (CTCAE 5.0 grading)**

| Grading | Before treatment | After treatment | Therapeutic effect | |
|---|---|---|---|---|
| | (Person, %) | (Person, %) | Effective | Remarkable effect/recovery |
| No | 0 (0%) | 3 (18.75%) | | |
| Grade 1 | 0 (0%) | 13 (81.25%) | 16 | 3 |
| Grade 2 | 16 (100.00%) | 0 (0%) | (100.00) | (18.75%) |

Note: the Chi-square test was used for comparing the differences before and after treatment, and the theoretical number of counting variables was < 10, as obtained by a Fisher accurate probability test, with p = 0.012.

(2) Adverse reactions: no adverse drug reactions such as allergy and infection were found during the treatment.

Example 4: Clinical experiment of the traditional Chinese medicine composition of the present disclosure in treatment of EGFR-TKIs related paronychia

### 1. Case 1

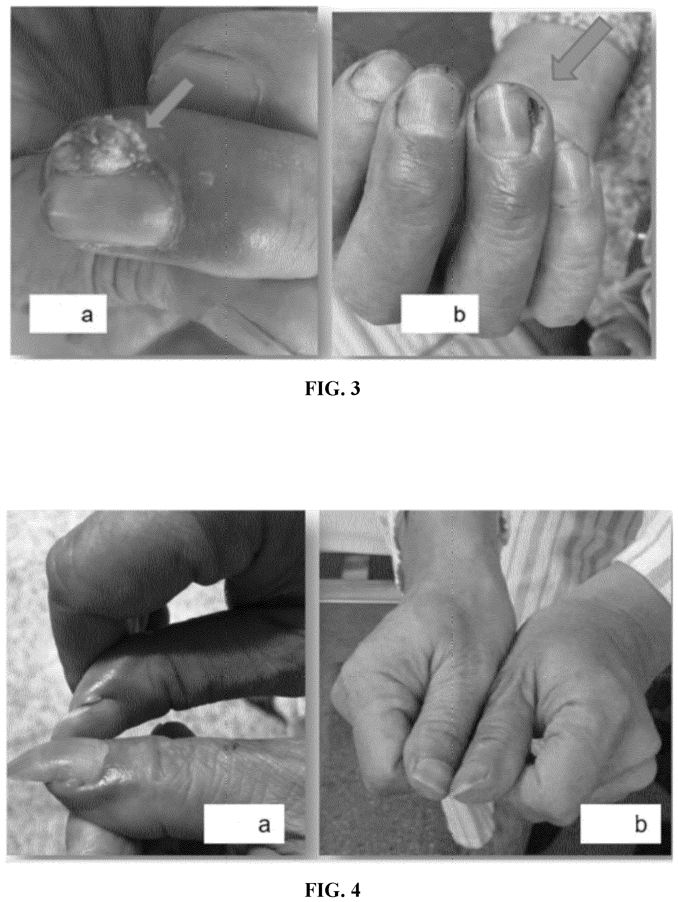

In case 1, the patient was Wu XX, a 49 years old female. In June, 2019, she was diagnosed as stage IV of lung adenocarcinoma. and had exon 19 mutation as detected by Gene detection. She took 40 mg QD of Afatinib, and paronychia occurred in her 2 months later, and gradually worsened with formation of granulation-like tissue (FIG. 3a). From August 3rd, 2019, she was soaked and washed with a topical skin rash-eliminating prescription twice a day, and after 1 week, the granulation tissue subsided, leaving only nail fold edema (FIG. 3b). The CTCEA grade was reduced from grade 2 to grade 1.

### 2. Case 2

In case 2, the patient was Guan XX, a 59 years old male. In August, 2019, he was diagnosed as stage IV of lung adenocarcinoma. and had exon 19 mutation as detected by Gene detection. He took 40 mg QD of Afatinib, and paronychia occurred in him 1 months later, and gradually worsened with nail fold edema accompanied with pain appeared on the lateral edges of both thumbs (FIG. 4a). From August 25th, 2019, he was soaked and washed with a topical skin rash-eliminating prescription twice a day, and after 1 week, the nail fold edema subsided without pain and discomfort, and he was basically recovered (FIG. 3b). The CTCAE score was reduced from grade 2 to recovery.

Example 9: Clinical experiment of the traditional skin rash composition of the present disclosure in treatment of EGFR-TKIs related skin rash

### 1. Case 1

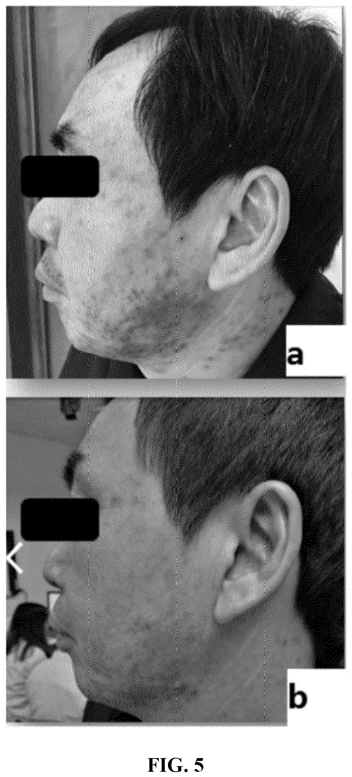

The patient was Huang XX, a 57-year-old male, who was diagnosed as left upper lobe adenocarcinoma with systemic multiple bone metastases after operation. From December, 2017 to October, 2018, he was treated by oral administration of Iressa, after more than 10 months, the tumor was progressed, and a severe skin rash was occurred after more than 4 months of administration. In November, 2018, Xia gene detection: Exon-21 (L858R mutation) and TP53 pathogenic variation were detected. In November, 2018, he had the first visit to our outpatient department, and was changed to oral administration of Tagrisso. In March, 2019, when the patient visited, the skin rash of him was progressively worsened. At that time, the skin rash on the face was mainly located on the left and right cheeks, was mainly papules and erythema, and had a dark red color. The total score of WoMo was 50, and the C part score of WoMo was 9 (FIG. 5a). After two weeks of intervention by administration of this protocol, the skin rash on the face was improved, and the facial papules and erythema obviously subsided. The total score of WoMo was 30, and the C part score of WoMo was 5 (FIG. 5b).

### 2. Case 2

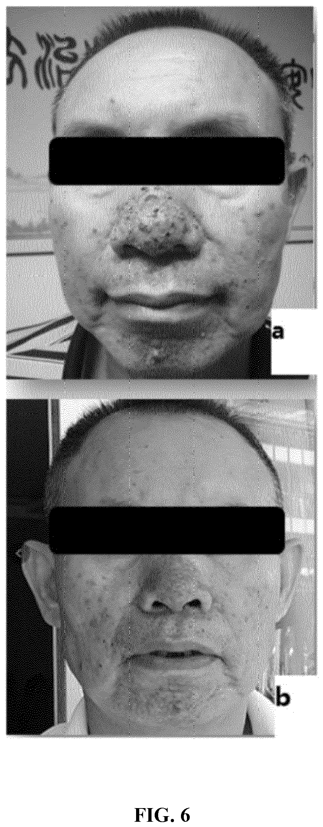

The patient was Shen XX, a 60 years old male. He was diagnosed as adenocarcinoma of the lower lobe of the left lung complicated with multiple M (IVa stage) in both lungs, bilateral supraclavicular bone, mediastinum and right hilum. From November 14th, 2018, he was given 7 courses of a platinum-pemetrexed (PP) regimen of chemotherapy + Avastin and immunotherapy. From May 9, 2019, he was given Alfatinib (30 mg) + Anlotinib (8mg), and the skin rash was occurred on the 16th day. The skin rash on the face of the patient was mainly located in the nose, cheeks of both sides and lower jaw, and was especially obvious in the nose. Meanwhile, erythema, papules and abscesses were also existed (FIG. 6a). The total score of WoMo was 60, and the C part score of WoMo was 13. After two weeks of external application of the skin rash-eliminating and itching-stopping prescription, the total score of WoMo was 30 and the C part score of WoMo was 7. The abscess of the nose was basically disappeared, leaving only a few papules and erythema (FIG. 6b).

### 3. Case 3

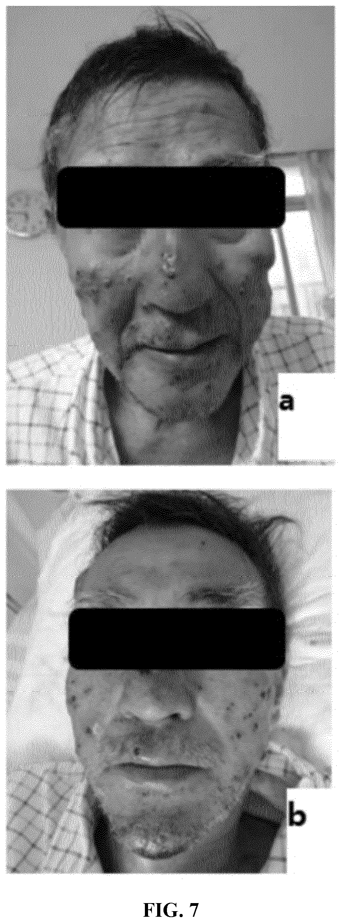

The patient was Chen XX, a 57 years old male. On August 20th, 2018, the CT examination of the patient showed a left upper lung occupying lesion. Pathological examination: Lung adenocarcinoma. EGFR detection: Exon 19 mutation. He was subsequently treated with the traditional Chinese medicine. On May 22, 2019, the CT showed that the upper left lung adenocarcinoma was larger than before, with liver metastasis. Then he took Alfatinib. On September 10th, 2019, the reexamination of CT showed that the upper left lung adenocarcinoma was reduced by 50%. Then he was subjected to left lung and liver metastases ablation, and the first small lesions of liver metastases appeared. Therefore, he was treated with " 1 g of pemetrexed disodium + 0.5 g of Bevacizumab" in the same period on October 15th, 2019 and November 14th, 2019. During the treatment, the patient developed severe skin rash, accompanied by cysts, abscesses, papules and erythema at the same time. The total score of WoMo was 80, and the C part score of WoMo was 10 (FIG. 7a). From October 27th, 2019, he began to be treated with the skin rash-eliminating and itching-stopping prescription. On the fourth day, the condition began to show obvious improvement, abscess began to heal, papules and cysts subsided, and the area of erythema was decreased. The total score of WoMo was 50, and the C part score of WoMo was 5 (FIG. 7b), so that the treatment effect was remarkable.

The examples described above are merely illustrative of several embodiments of the present disclosure, the description of them is more specific and detailed, but cannot be construed as limiting the scope of the present disclosure accordingly. It should be noted that, several variations and modifications can be made by those of ordinary skills in the art, under the premise of not departing from the concept of the present disclosure, and these variations and modifications all fall within the claimed scope of the present disclosure. Therefore, the scope of protection of the present disclosure shall be determined by the appended claims.

## Claims

1. A traditional Chinese medicine composition, comprising therapeutically effective amounts of the following components: *Lonicera japonica, Taraxacum mongolicum, Sophora flavescens, Kochiae fructus, dictamni cortex, moutan cortex, Phellodendri Chinensis Cortex,* and *Menthae Haplocalycis Herba;* and
preferably, the traditional Chinese medicine composition is a traditional Chinese medicine composition for treating EGFR-TKIs related skin rash.

2. The traditional Chinese medicine composition according to claim 1, comprising 5-40 parts of *Lonicera japonica,* 5-50 parts of *Taraxacum mongolicum,* 3-50 parts of *Sophora flavescens,* 5-50 parts of *Kochiae fructus,* 3-50 parts of *dictamni cortex,* 5-50 parts of *moutan cortex,* 5-50 parts of *Phellodendri Chinensis Cortex,* and 2-30 parts of *Menthae Haplocalycis Herba.*

3. The traditional Chinese medicine composition according to claim 1, comprising 15 parts of *Lonicera japonica,* 15 parts of *Taraxacum mongolicum,* 9 parts of *Sophora flavescens,* 15 parts of *Kochiae fructus,* 10 parts of *dictamni cortex,* 12 parts of *moutan cortex,* 12 parts of *Phellodendri Chinensis Cortex,* and 6 parts of *Menthae Haplocalycis Herba.*

4. Use of the traditional Chinese medicine composition according to any one of claims 1-3 in preparation of a formulation for treating EGFR-TKIs related skin rash.

5. Use of the traditional Chinese medicine composition according to any one of claims 1-3 in preparation of a formulation for treating EGFR-TKIs related paronychia.

6. The use of the traditional Chinese medicine composition according to claim 4 or 5, wherein the formulation is a liniment, water aqua, decoction, granule, paste, mistura, aerosol, gel, ointment, facial mask, cream or unguent.

7. A method for treating EGFR-TKIs related skin rash or paronychia, comprising administering the traditional Chinese medicine composition according to any one of claims 1-3 to a patient in need thereof, such as a human.
